# EUROPEAN PATENT SPECIFICATION

(11) **EP 3 927 397 B1**
(45) Date of publication and mention of the grant of the patent: **11.12.2024**
(21) Application number: 20705122.8
(22) Date of filing: 21.02.2020
(51) Int. Cl.: A61M 5/20, A61M 5/32, A61M 5/34, A61M 5/24

(54) **CARTRIDGE BASED AUTO-INJECTOR**
KARTUSCHENBASIERTER AUTOMATISCHER INJEKTOR
AUTO-INJECTEUR À BASE DE CARTOUCHE

(30) Priority: 21.02.2019 GB 201902355
(43) Date of publication of application: 29.12.2021
(73) Proprietor: Sensile Medical AG, 4600 Olten (CH)
(72) Inventor: DUNNE, Stephen, Suffolk IP14 1DT (GB)
(74) Representative: Bardehle Pagenberg Partnerschaft mbB Patentanwälte Rechtsanwälte
(86) International application number: PCT/EP2020/054704
(87) International publication number: WO 2020/169842

(56) References cited:
- WO-A1-2012/072552
- WO-A1-2015/121655
- WO-A2-2012/022810

## Description

### CARTRIDGE BASED AUTO-INJECTOR

The present invention is an auto-injector for the administration and self-administration of drugs by subcutaneous, intramuscular or intra-dermal injection and for drug solutions or suspensions. This application describes an injection system that may be used by the patient for self-injection or by a medical professional.

Auto-injectors automate the injection stroke alleviating the need for the patient or professional to actuate the plunger rod.

WO2015/121655 discloses an injector assembly comprising a casing within which a collapsible medicament container, a biasing means consisting of a first and a second spring for applying a first and a second pressure continuously to a piston and consequently to the container. The injector assembly according to prior art further comprises a shuttle with needles at each of its sides and a removable needle guard. Prior to use of the injector according to prior art patent application WO2015/121655, the needle guard has to be removed. In use, the first spring acts between second spring and the piston to apply a first force to the piston in such a way that when the first spring is compressed, it provides a biasing force between the second spring and the piston, thereby the collapsible medicament container is pushed by the piston against the needle, which perforates the septum. The injector assembly is configurated to pressurize the medicament with different viscosity under different pressure during the storage.

The invention is defined by the appended claims. In an example, an auto-injector comprises in an outer casing that holds a standard cartridge comprising a barrel with a drug contents being contained by a movable plunger or stopper at one end, and a seal or septum and metal crimp or cap to hold said septum in place at the other end. A biased means that pushes against the movable plunger or stopper of the cartridge thus pressurizing the drug contents. A needle shuttle is held within casing comprises two needles that are attached and connected to each other, the first needle being the injection needle and the second needle being the septum needle. A means or shoulder retains the cartridge in its correct position when inserted and prevents it from being pushed against the needle shuttle. A needle guard is connected to the casing, being biased outwards by a spring or the like such that the needle is always covered by the needle guard, thus hiding the needle before and after the injection.

The auto-injector according the present invention may further comprise a needle guard locking mechanism that prevents the needle guard from being pushed back a second time, thus preventing needle stick injuries after use.

The auto-injector may further comprise an indicator. The indicator may be in form of an indicator rod that is attached to the biased means or the movable plunger or stopper and thus moves with it, thus allowing to indicate the progress of the injection progress. The indicator rod may be connected and used to operate any other indicator such as a rotary indicator or an electronic indicator or a connectivity device.

In one embodiment, the indicator extends beyond the rear casing to a distance determined by the fill volume and enabling the user to monitor the status of the injection.

In a preferred embodiment, the indicator rod has a closed end and contains the spring within. The outer casing comprises a portion that allows to monitor the progress and status of the injection progress, for example a cap which is transparent or has viewing windows. If the drug volume is fully injected, the indicator is no longer visible indicating end of injection. Placing the spring inside the indicator has the advantage that the spring can be longer than if the indicator is placed inside the spring or the device may be shorter. A longer spring means a higher mean force which is advantageous for viscous drugs.

The invention further relates to a modular designed auto injector which is composed of several sub-assemblies or modules. For ease of assembly, the modular auto-injector comprises three sub-assemblies, namely a cartridge, a power source and indicator sub-assembly and a needle and needle guard sub-assembly.

The cartridge sub- assembly comprises a barrel, a drug volume that is bounded by a plunger and a crimped septum.

The power source and indicator assembly comprises a biased means or spring and an indicator rod that is held within a rear casing. The invention allows for the cartridge to have a variable fill volume when assembled with the power source and indicator sub-assembly.

In order to indicate the status and progress of the injection process, the indicator rod may be connected to either the biased means or spring or to the movable plunger or stopper upon assembly of the device in a way that it moves with it.

If the biased means is a spring it may further comprise a spring locking member. In this case the power source and indicator assembly may further comprise a second weaker spring to pressurize the cartridge drug contents prior to injection while the first spring is locked by the spring locking member. The second spring also avoids a pressure pulse when the second spring is released.

The needle and needle guard sub-assembly comprises a front casing holding a needle shuttle with an injection needle and a septum needle, the shuttle being held within the casing , a needle guard for preventing needle stick injuries that is biased outwards by a spring, a locking mechanism that prevents the needle guard from being pushed back a second time, and a means to retain the cartridge in its correct position when inserted.

The injection needle and the septum needle may have the same diameter or gauge or have different diameters or gauges. They may have the same type of bevel tip or different types of bevel tip. For example, to minimize injection time the septum needle may be of larger diameter or smaller gauge than the injection needle. For simplicity a single needle may be used with two sharp ends or bevels. Different needle gauges and/or bevels can be used to ensure the injection needle penetrates the injection site before the septum needle penetrates the septum to ensure a full injection. The interchange of the injection needle allowing adaption for subcutaneous, intramuscular or intra-dermal injection.

The modular auto-injector may be assembled by an easy two-step process such as placing the cartridge into the needle and needle guard sub-assembly followed by connecting it with the power source and indicator sub-assembly in a way that the drug contents in the cartridge are pressurized. If the device is intended for single use, any connection means to lock the sub-assemblies of the modular auto-injector together may be used, such as a snap fit.

In a preferred embodiment, the modular auto-injector can be re-assembled or is multiple use. In this case the needle and needle guard assembly and the power source and indicator assembly may be connected by a a bayonet fit or a screw or are hinged together to allow a reversible connection. In addition, the biased means of the power source and indicator assembly allows for a multiple use such as an electric motor, a liquified gas cannister or a spring with means to re-compress the spring. Finally, the needle and needle guard assembly comprises a seal and a removable needle cap that always keep the needles and the cartridge septum sterile.

The embodiments of the auto-injector according to the present invention may be assembled under sterile conditions or may be submitted to terminally sterilization after assembly.

If the drug in the cartridge is a small molecule (non-biologic) the assembled device may be terminally sterilized in a blister or pouch using gas, vapor or radiation such as gamma ray, e-beam or any other.

When sterilizing the device with sensitive drug contents such as biologic drugs, the blister/pouch with the assembled device has at least a portion made from gas permeable material. The device may have gas pathways to assure that the needles and the external surface of the cartridge septum are sterilized. The device can then be placed in a gaseous environment such as ethylene oxide or nitrogen dioxide for sterilization.

To prevent the gas affecting sensitive drug contents, the cartridge assembly may preferably have gas impermeable components, in particular plunger/stopper and septum/cap. The gas used should preferably not be at elevated temperatures and preferably below body temperature and preferably below 36°C.

The device may be supplied as a ready-to-use medical device or supplied pre-assembled as a combination product ("kit") for self-assembly by the user.

Sterilisation may take place before assembly or after assembly in which case it is terminally sterilized. If sterilized before assembly only the needle and guard sub-assembly or module 182 need be sterilized. The assembled device may be terminally sterilized in a blister or pouch. A needle cap and maybe a seal is required if the device is sterilized outside a blister. If the device is assembled by the user, the cartridge can be supplied in a blister or with no blister in which case the septum needs to be wiped with ethanol before device assembly.

Table 1 shows an overview of the different options as regards sterilization, options 3 to 6 allowing for re-assembly.

**Table 1**

| | | Seal 128 essential? | Cap 126 essential? | Gas permeable elastomer essential? |
|---|---|---|---|---|
| 1. | Fully assembled with cartridge IN BLISTER | NO | NO | N/A |
| 2. | Fully assembled with cartridge OUTSIDE BLISTER | NO | YES | Yes for gas No for radiation |
| 3. | Sub-assembly 182 IN BLISTER | NO | NO | N/A |
| 4. | Sub-assembly 182 OUTSIDE BLISTER | YES | YES | Yes for gas No for radiation |
| 5. | Sub-assembly 182 assembled WITH cartridge 181 IN BLISTER | NO | NO | N/A |
| 6. | Sub-assembly 182 assembled WITH cartridge 181 OUTSIDE BLISTER | NO | YES | Yes for gas No for radiation |

The auto-injector according to the present invention may be used with drug suspensions or solutions and API's or biologics. If the suspension is unstable with settling tendencies the cartridge needs to be shaken before use to re-mix.

The modular design has the advantage that each sub-assembly may be produced separately under conditions individually adapted. It further allows for flexibility in production and allows different auto injectors to share modules. The modular design also allows for part exchangeability which bears the additional advantage is that modules or sub-assemblies can be interchanged to change the characteristics of the auto-injector. For instance, by changing the needle and hub and needle guard assembly the device can be changed from a subcutaneous auto-injector to an intramuscular or intra-dermal auto-injector. It also allows for the power source module to be a reusable device. The modular designed auto-injector does also allow the cartridge to be exchanged, either after it has been used or if its shelf-life has expired.

The present invention allows for the use of industry standard injection cartridge and manufacture auto-injectors with a dry needle during storage and with various other advantages.

With reference to the figures, specific embodiments of the present invention are described hereinafter:

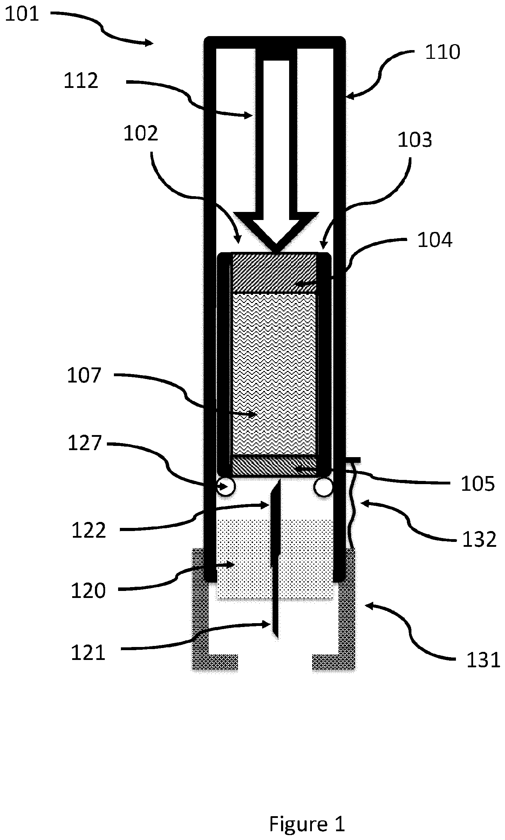

Figure 1 shows an auto injector 101 according to the present invention. A standard cartridge 102 has barrel 103 with drug solution or suspension 107 within contained by a movable plunger or stopper 104 at one end and a rubber seal or septum and metal crimp 105 at the other end. The cartridge 102 is held in casing 110. A biased means 112 pushes against the stopper 104 pressurizing contents 107. A needle shuttle 120 is held within casing 110. Shuttle 120 has two needles 121 and 122 attached and connected to each other. Needle 121 is the injection needle and needle 122 is the septum needle. A means 127 retains cartridge 103 in its correct position when inserted and prevents it from being pushed against the shuttle. A needle guard 131 and spring 132 are held to casing 110. Needle guard 131 has the purpose of preventing needle stick injuries after injection.

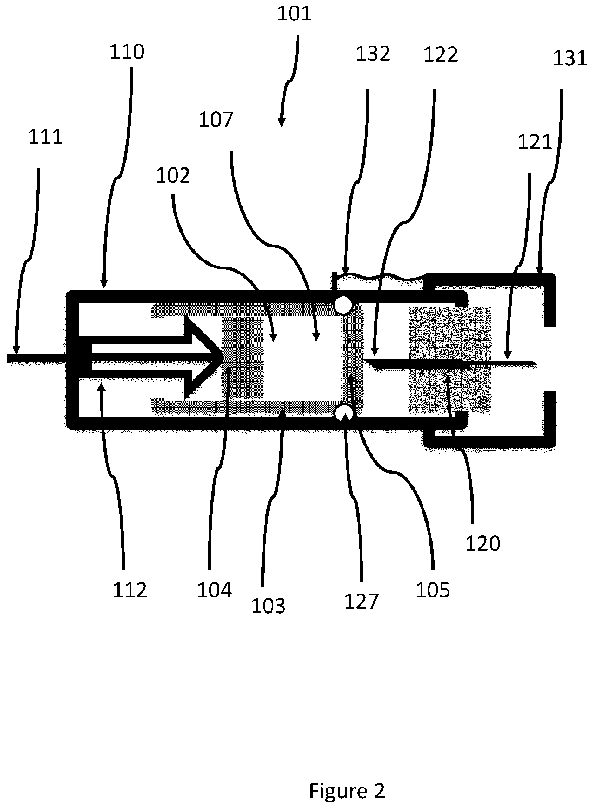

Figure 2 shows the auto injector 101 with an indicator rod 111 being attached to stopper 104.

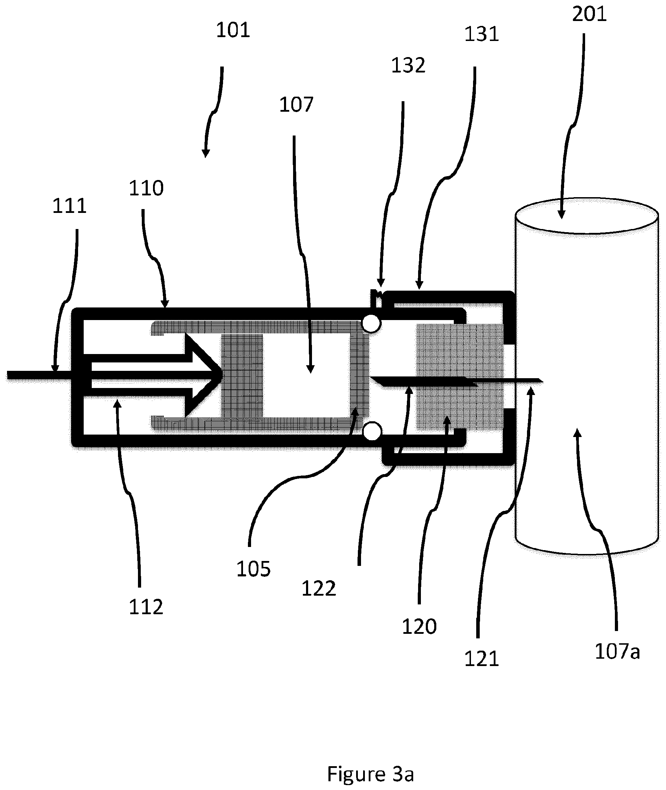

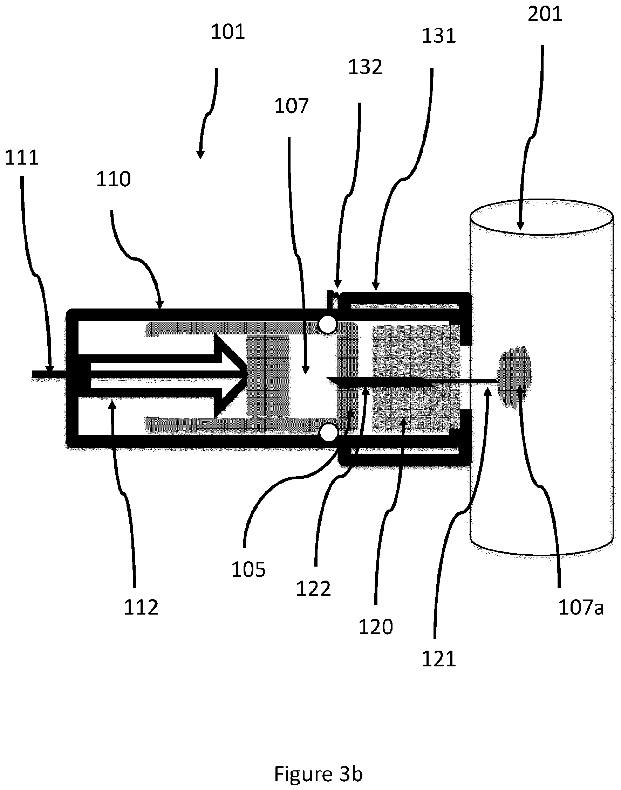

Figures 3a and 3b show the auto-injector 101 during injection. Auto-injector 101 is pressed against injection site 201. Needle 121 is inside the injection site 201 and delivering drug solution or suspension 107a as a bolus. Needle guard 131 has been pushed against injection site 201 and been pushed back relative to casing 110 against spring 132 pushing back shuttle 120. Needle 122 has pierced septum 105 and the pressurized contents 107 is flowing into injection site 201 via needles 121 and 122 and delivering a bolus 107a. The indicator 111 is moving forward showing injection progress. To ensure a complete injection, the injection needle penetrates the injection site (Fig 3a) before the septum needle penetrates the septum (Figure 3b).

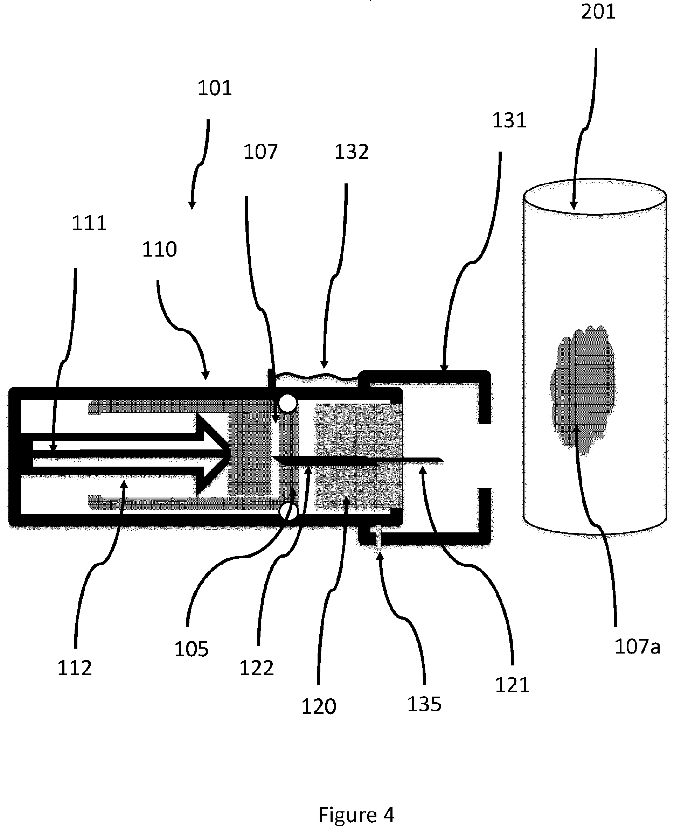

In Figure 4 the auto injector 101 has finished injecting, the needle guard 131 has been pushed back by spring 132 and been locked into position by locking mechanism 135.

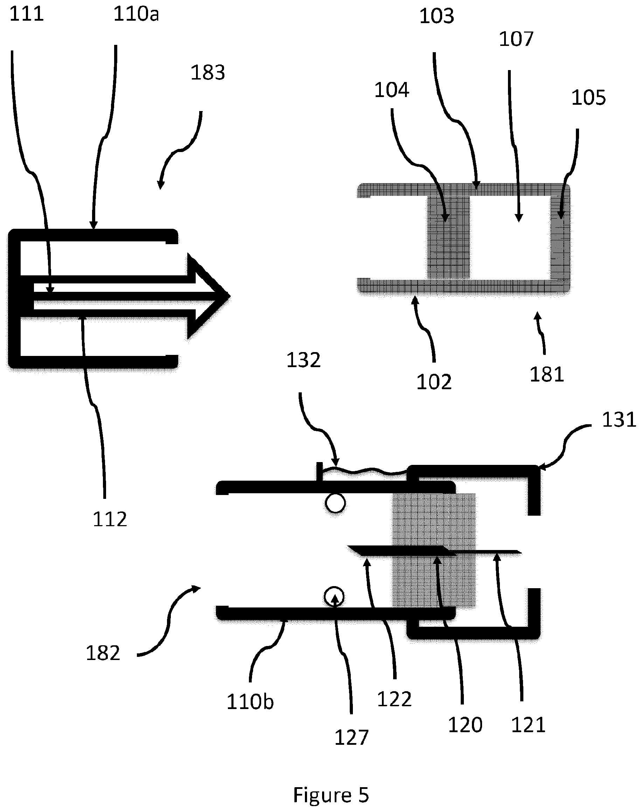

Figure 5 shows a modular auto injector according to the present invention which is made up by three sub-assemblies: A cartridge sub-assembly 181, a needle and needle guard sub-assembly 182 and a power source and indicator sub-assembly 183.

Cartridge sub-assembly 181 has a cartridge 102 with barrel 103, drug volume 107 held by plunger 104 and septum 105.

Power source and indicator sub-assembly 183 has a biased means or spring 112 and indicator rod 111 held within casing 110a.

Needle and needle guard sub-assembly 182 has a casing 110b holding a needle shuttle 120 with needles 121 and 122. A needle guard 131 for preventing needle stick injuries is biased outwards by spring 132. Module 182 may be supplied sterilized. It also has a guard locking mechanism a shoulder 127 that retains the cartridge 102 in its correct position when inserted not shown.

Module 182 may contain a needle for subcutaneous, intramuscular or intra-dermal injections.

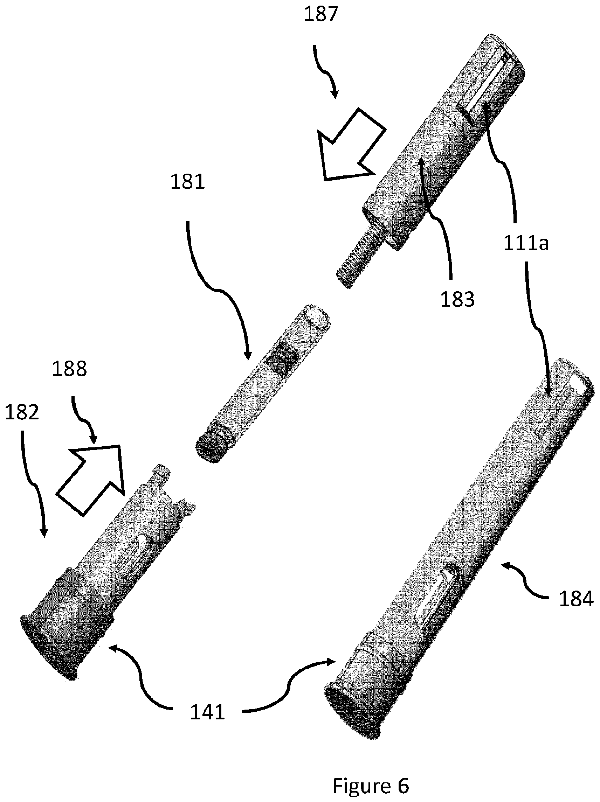

Figure 6 shows a 3-dimensional view of the modular auto injector. Cartridge 181 is placed into sub-assembly 182 followed by sub-assemblies 183 and 182 being locked together in the directions of arrows 187 and 188 pressurizing the drug contents in cartridge 181.

The assembled auto injector 184 is shown with an additional indicator cap and indicator knob 111a which is attached to the indicator rod 111 (not shown) and an additional front cap 141 which are optional features.

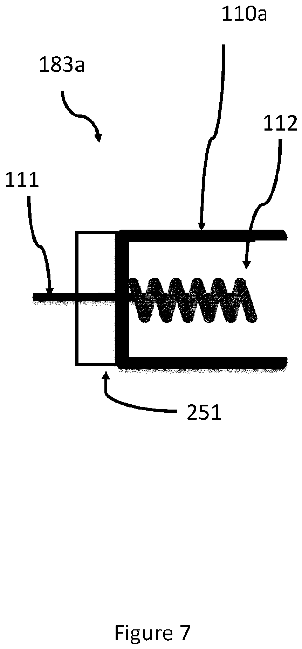

In Figure 7 sub-assembly 183a has a locking member 251 holding spring 112 compressed and back by the indicator rod 111 for ease of device assembly. The locking member 251 is slowly removed after assembly of the device freeing spring 112 to slowly compress cartridge contents and avoid a pressure pulse. The locking member 251 may be a nut on a threaded indicator rod or any other mechanism.

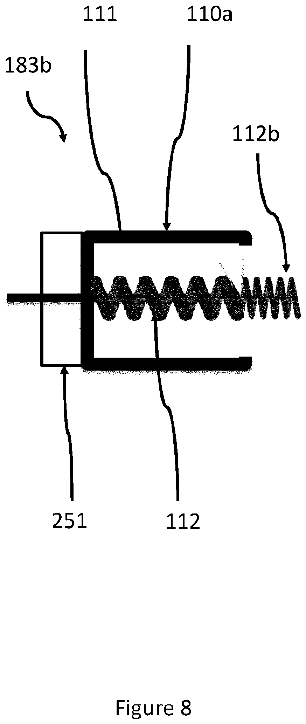

In Figure 8 sub-assembly 183b has an additional spring 112b which is weaker than spring 112 that compresses the drug solution in the cartridge after the device is assembled. Before injecting the locking device 251 is removed so that spring 112 can fully pressurize the cartridge contents.

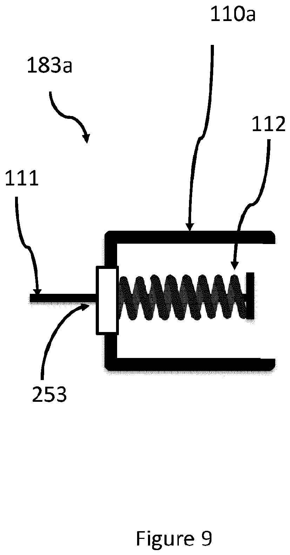

In Figure 9 another embodiment is shown. A latch 253 holds the spring 112 compressed and holds it back for easy assembly. Latch 253 releases the spring when the device is assembled by outwards axial movement of the indicator 111 compressing the drug contents.

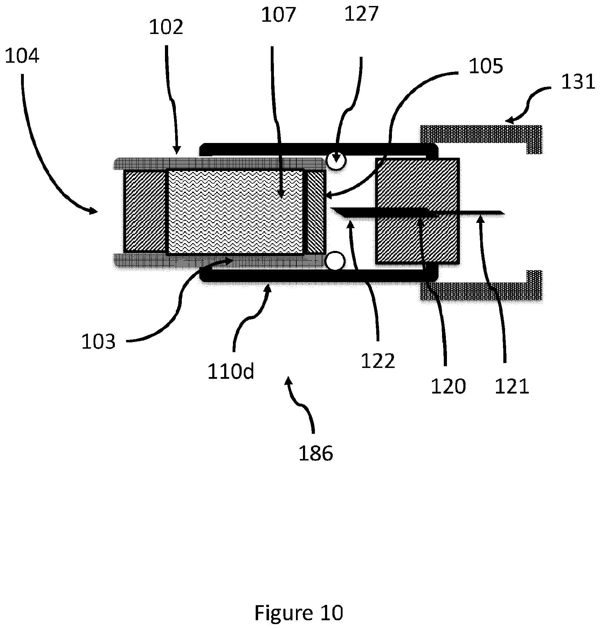

Figure 10 shows another embodiment of the invention. Module 186 is a combination of sub-assemblies 181 and 182 and has the cartridge 102 supplied to the user with the needles 121 and 122 attached and is held in place with the needles 121 and 122.

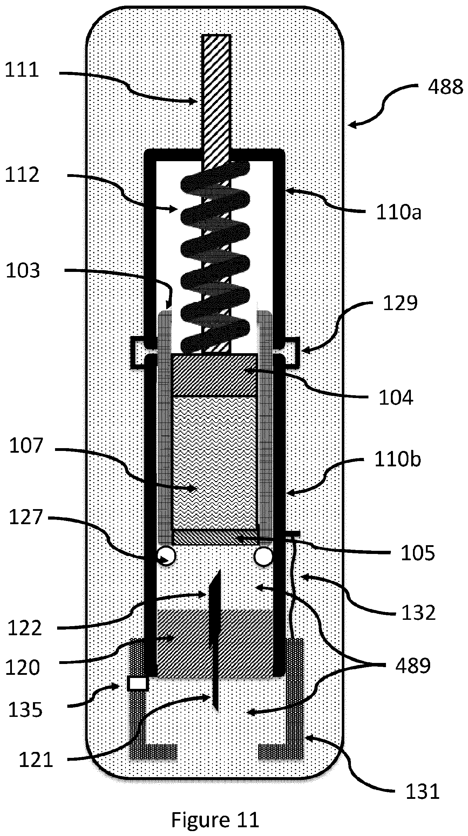

Figure 11 shows an assembled and ready-to-use auto-injector that is being placed in a blister or pouch 488 and submitted to a gaseous environment for terminal sterilization, 489 indicates the sterilized area.

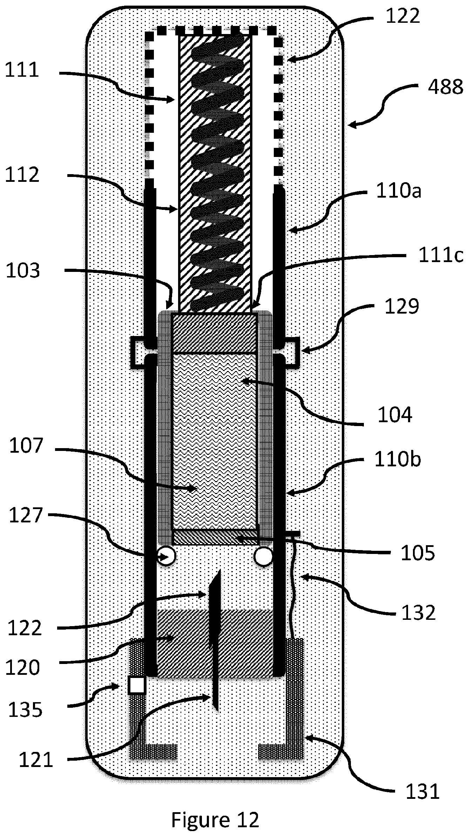

Figure 12 shows an assembled and ready-to-use auto-injector wherein the indicator 111 contains the spring 112 within and an cap 122 that allows the observation of the injection progress.

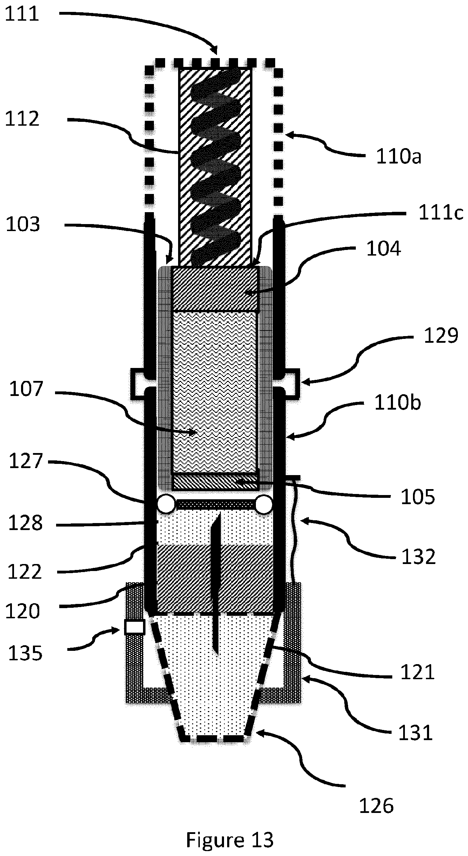

Figure 13 shows an assembled and ready-to-use auto-injector comprising an additional seal 128 and a elastomeric needle cap 126 to keep the needles sterile. The use of cap 126 and optionally seal 128 allows for terminal sterilization without the use of a blister. For easier removal from needle guard 131 the cap 126 is made from an elastomeric material such as rubber.

A preferred embodiment of the present invention is a ready-to-use modular auto-injector that comprises
- a cartridge sub-assembly 181 comprising a barrel 103, a drug volume 107 held by a movable plunger or stopper 104 and a crimped septum 105,
- a power source and and indicator sub-assembly 183 comprising a rear casing 110a holding a biased means 112 and an indicator rod 111,
- a needle and needle guard sub-assembly 182 comprising a front casing 110b holding a needle shuttle 120 with an injection needle 121 and a septum needle 122, a needle guard 131 and spring 132, a locking mechanism 135 that prevents the needle guard 131 from being pushed back a second time, and a means or shoulder 127 to retain the cartridge 102 in its correct position when the device is assembled, where the assembled device is placed in a blister or pouch 488 and terminally sterilized.

Another preferred embodiment of the present invention is a ready-to-use and re-usable modular auto-injector that comprises
- a cartridge sub-assembly 181 comprising a barrel 103, a drug volume 107 held by a movable plunger or stopper 104 and a crimped septum or cap 105,
- a and and indicator assembly 183 comprising a rear casing 110a n indicator rod 111 that contains a spring 112 within,
- a needle and needle guard sub-assembly 182 comprising a front casing 110b holding a needle shuttle 120 with an injection needle 121 and a septum needle 122, a needle guard 131 and spring 132, a locking mechanism 135 that prevents the needle guard 131 from being pushed back a second time, a shoulder 127 to retain the cartridge 102 in its correct position when inserted and a seal 128 and an removable elastomeric needle cap 126 to keep the needles sterile, and
- reversible connection means 129 allowing a re-assembly or user assembly of the device,
- where assembly 182 is supplied sterilized and ready for use either in a blister and/or supplied with a seal 128 and an needle cap 126.

## Claims

1. An auto-injector (101) comprising an outer casing (110) holding a standard cartridge (102) consisting of
a barrel (103) with a drug content (103) being contained by a movable plunger (104) at one end and a seal and metal crimp (105) at the other end,
a biased means (112) that pushes against the movable plunger (104) of the cartridge (102) pressurizing the drug content (103),
a needle shuttle (120) being held within the casing (110) comprising two needles (121, 122) that are attached and connected to each other,
a first needle (121) being the injection needle and a second needle (122) being the septum needle,
a means (127) to retain the cartridge in its correct position when inserted,
**characterized in that** the auto-injector further comprises
a needle guard (131) and a spring (132) held to the outer casing (110), wherein the needle guard (131) is being biased outwards by the spring (132), and wherein the needle guard is configured such that during injection, when the auto-injector (101) is pressed against an injection site (201), the needle guard (131) is pushed against the injection site (201), the needle guard (131) is pushed back relative to the casing (110) against the spring (132) and thereby pushes back the needle shuttle (120) to pierce the seal and when the auto injector (101) has finished injecting, the needle guard (131) is pushed back by spring (132) such that the first needle (121) is always covered by the needle guard (131).

2. An auto-injector (101) according to claim 1, further comprising a locking mechanism (135) that prevents the needle guard (131) from being pushed back a second time.

3. An auto-injector (101) according to claim 1 or claim 2, further comprising an indicator rod (111) that is attached to the movable plunger (104).

4. An auto-injector (101) according to claim 3 wherein the indicator rod (111) has a closed end containing the biased means (112) in form of a spring within, the outer casing (110) comprising a transparent portion that allows to monitor the progress and status of the injection progress.

5. An auto-injector (101) according to any of the preceding claims wherein the auto-injector is a modular auto-injector which comprises three sub-assemblies (181, 182, 183), namely a cartridge sub-assembly (181),
a power source and indicator assembly (183), and
a needle and needle guard assembly (182).

6. An auto-injector (101) according to claim 5 wherein the cartridge sub-assembly (181) comprises a barrel (103) and a drug volume (107) being bounded by a plunger (104) and a crimped septum (105).

7. An auto-injector (101) according to one of claims 5 to 6 wherein the power source and indicator sub-assembly (183) comprises a biased means (112) and an indicator rod (111) that is held within a rear casing.

8. An auto-injector (101) according to claim 7, wherein the indicator rod (111) may be connected to the movable plunger (104) in a way that it moves with it.

9. An auto-injector (101) according to any of claims 6 to 8, wherein the biased means (112) of the power source and indicator sub-assembly (183) is a spring (112) that further comprises a spring locking member (251).

10. An auto-injector (101) according to claim 9, wherein the power source and indicator sub- assembly (183) further comprises an additional spring (112b) which is weaker than the spring (112) to pressurise the cartridge drug contents (107) prior to injection while the spring (112) is locked by the spring locking member (251).

11. An auto-injector (101) according to any of claims 5 to 10, wherein the needle and needle guard sub-assembly (182) comprises a casing (110b) holding a needle shuttle (120) with an injection needle (121) and a septum needle (122), a needle guard (131) and spring (132), a locking mechanism (135) that prevents the needle guard (131) from being pushed back a second time, and a shoulder (127) to retain the cartridge (102) in its correct position when inserted.

12. An auto-injector (101) according to claim 10 or claim 11 for re-assembly, wherein the needle and needle guard sub-assembly (182) further comprises a seal (128) and a needle cap (126), the biased means of the power source and indicator sub-assembly is an electric motor or a liguified gas cannister or a spring with means to re-compress the spring, and the needle and needle guard sub-assembly (182) and the power source and indicator sub-assembly (183) are reversibly connected by a bayonet fit or a screw or are hinged together.

13. Process for the assembly of a modular auto-injector according to claim 5 to 12 including the following steps:
inserting a cartridge (102) into a needle and needle guard sub-assembly (182), and
connecting a power source and indicator sub-assembly (183) via connection means to the needle and needle guard sub-assembly (182) with inserted cartridge (102) in a way that the drug contents in the cartridge is pressurized.

14. The process according to claim 13, further comprising the steps of inserting the pre-assembled device in a blister or pouch (488), and submitting the blister or pouch (488) to terminal sterilization.

15. The assembly process according to claim 14, wherein the terminal sterilization is carried out by ethylene oxide or nitrogen dioxide.

## Patentansprüche

1. Autoinjektor (101), umfassend ein äußeres Gehäuse (110), das eine Standardkartusche (102) hält, die aus einem Zylinder (103) mit einem Arzneimittelinhalt (103) besteht, der an einem Ende von einem beweglichen Kolben (104) und am anderen Ende von einer Dichtung und einem Metallcrimp (105) enthalten ist,
ein vorgespanntes Mittel (112), das gegen den beweglichen Kolben (104) der Kartusche (102) drückt und den Arzneimittelinhalt (103) unter Druck setzt,
ein Nadelgreifer (120), der in dem Gehäuse (110) gehalten wird und zwei Nadeln (121, 122) umfasst, die aneinander angebracht und miteinander verbunden sind,
wobei eine erste Nadel (121) die Injektionsnadel ist und eine zweite Nadel (122) die Septumnadel ist,
ein Mittel (127), um die Kartusche in ihrer korrekten Position zu halten, wenn sie eingeführt wird,
**dadurch gekennzeichnet, dass** der Autoinj ektor ferner Folgendes umfasst:
einen Nadelschutz (131) und eine Feder (132), die an dem äußeren Gehäuse (110) gehalten werden, wobei der Nadelschutz (131) durch die Feder (132) nach außen vorgespannt wird und wobei der Nadelschutz so konfiguriert ist, dass während der Injektion, wenn der Autoinjektor (101) gegen eine Injektionsstelle (201) gedrückt wird, der Nadelschutz (131) gegen die Injektionsstelle (201) gedrückt wird, der Nadelschutz (131) relativ zu dem Gehäuse (110) gegen die Feder (132) zurückgedrückt wird und dadurch den Nadelgreifer (120) zurückdrückt, um die Dichtung zu durchstechen, und wenn der Autoinjektor (101) die Injektion beendet hat, der Nadelschutz (131) durch die Feder (132) zurückgedrückt wird, so dass die erste Nadel (121) immer von dem Nadelschutz (131) bedeckt ist.

2. Autoinjektor (101) nach Anspruch 1, ferner umfassend einen Verriegelungsmechanismus (135), der verhindert, dass der Nadelschutz (131) ein zweites Mal zurückgedrückt wird.

3. Autoinjektor (101) nach Anspruch 1 oder Anspruch 2, ferner umfassend eine Anzeigestange (111), die an dem beweglichen Kolben (104) angebracht ist.

4. Autoinjektor (101) nach Anspruch 3, wobei die Anzeigestange (111) ein geschlossenes Ende aufweist, das das vorgespannte Mittel (112) in Form einer Feder enthält, wobei das äußere Gehäuse (110) einen transparenten Abschnitt umfasst, der es ermöglicht, den Fortschritt und den Status des Injektionsfortschritts zu überwachen.

5. Autoinjektor (101) nach einem der vorhergehenden Ansprüche, wobei der Autoinjektor ein modularer Autoinjektor ist, der drei Unterbaugruppen (181, 182, 183) umfasst, nämlich eine Kartuschenunterbaugruppe (181), eine Stromquellen- und Anzeigebaugruppe (183) und eine Nadel- und Nadelschutzbaugruppe (182).

6. Autoinjektor (101) nach Anspruch 5, wobei die Kartuschenunterbaugruppe (181) einen Zylinder (103) und ein Arzneimittelvolumen (107) umfasst, das von einem Kolben (104) und einem gecrimpten Septum (105) begrenzt wird.

7. Autoinjektor (101) nach einem der Ansprüche 5 bis 6, wobei die Stromquellen- und Anzeigeunterbaugruppe (183) ein vorgespanntes Mittel (112) und eine Anzeigestange (111) umfasst, die in einem hinteren Gehäuse gehalten wird.

8. Autoinjektor (101) nach Anspruch 7, wobei die Anzeigestange (111) mit dem beweglichen Kolben (104) so verbunden sein kann, dass sie sich mit ihm bewegt.

9. Autoinjektor (101) nach einem der Ansprüche 6 bis 8, wobei das vorgespannte Mittel (112) der Stromquellen- und Anzeigeunterbaugruppe (183) eine Feder (112) ist, die ferner ein Federverriegelungselement (251) umfasst.

10. Autoinjektor (101) nach Anspruch 9, wobei die Stromquellen- und Anzeigeunterbaugruppe (183) ferner eine zusätzliche Feder (112b) umfasst, die schwächer ist als die Feder (112), um den Kartuschenarzneimittelinhalt (107) vor der Injektion unter Druck zu setzen, während die Feder (112) durch das Federverriegelungselement (251) verriegelt ist.

11. Autoinjektor (101) nach einem der Ansprüche 5 bis 10, wobei die Nadel- und Nadelschutzunterbaugruppe (182) ein Gehäuse (110b) umfasst, das einen Nadelgreifer (120) mit einer Injektionsnadel (121) und einer Septumnadel (122), einen Nadelschutz (131) und eine Feder (132), einen Verriegelungsmechanismus (135), der verhindert, dass der Nadelschutz (131) ein zweites Mal zurückgedrückt wird, und eine Schulter (127) hält, um die Kartusche (102) in ihrer korrekten Position zu halten, wenn sie eingeführt wird.

12. Autoinjektor (101) nach Anspruch 10 oder Anspruch 11 zur Wiedermontage, wobei die Nadel- und Nadelschutzunterbaugruppe (182) ferner eine Dichtung (128) und eine Nadelkappe (126) umfasst, das vorgespannte Mittel der Stromquellen- und Anzeigeunterbaugruppe ein Elektromotor oder ein Flüssiggaskanister oder eine Feder mit Mitteln zum Wiederzusammendrücken der Feder ist und die Nadel- und Nadelschutzunterbaugruppe (182) und die Stromquellen- und Anzeigeunterbaugruppe (183) durch einen Bajonettverschluss oder eine Schraube reversibel verbunden oder gelenkig miteinander verbunden sind.

13. Verfahren zur Montage eines modularen Autoinj ektors nach Anspruch 5 bis 12, umfassend die folgenden Schritte:
Einführen einer Kartusche (102) in eine Nadel- und Nadelschutzunterbaugruppe (182) und Verbinden einer Stromquellen- und Anzeigeunterbaugruppe (183) über Verbindungsmittel mit der Nadel- und Nadelschutzunterbaugruppe (182) mit eingeführter Kartusche (102), so dass der Arzneimittelinhalt in der Kartusche unter Druck gesetzt wird.

14. Verfahren nach Anspruch 13, ferner umfassend die Schritte des Einführens der vormontierten Vorrichtung in einen Blister oder Beutel (488) und des Unterziehens des Blisters oder Beutels (488) einer Endsterilisation.

15. Montageverfahren nach Anspruch 14, wobei die Endsterilisation durch Ethylenoxid oder Stickstoffdioxid durchgeführt wird.

## Revendications

1. Un auto-injecteur (101) comprenant une enveloppe externe (110) logeant une cartouche standard (102), constitué par un cylindre (103) avec un contenu médicamenteux (103) qui est enfermé par un piston mobile (104) à une extrémité et par un joint et un sertissage en métal (105) à l'autre extrémité,
un moyen sollicité (112) qui appuie contre le piston mobile (104) de la cartouche (102) en mettant sous pression le contenu médicamenteux (103),
une navette à aiguilles (120) qui est logée à l'intérieur de l'enveloppe (110), comprenant deux aiguilles (121, 122) qui sont fixées et reliées l'une à l'autre,
une première aiguille (121) étant l'aiguille d'injection et une seconde aiguille (122) étant l'aiguille de septum, un moyen (127) pour maintenir la cartouche dans sa position correcte lorsqu'elle est insérée,
**caractérisé en ce que** l'auto-injecteur comprend en outre un protège-aiguille (131) et un ressort (132) logé par l'enveloppe externe (110), le protège-aiguille étant sollicité vers l'extérieur par le ressort (132), et le protège-aiguille étant configuré de telle manière que pendant l'injection, lorsque l'auto-injecteur (101) est appuyé contre un site d'injection (201), le protège-aiguille (131) soit poussé contre le site d'injection (201), le protège-aiguille (131) soit repoussé en arrière par rapport à l'enveloppe (110) à l'encontre du ressort (132) et repousse ainsi en arrière la navette à aiguilles (120) de façon à percer le joint et, lorsque l'auto-injecteur (101) a achevé l'injection, le protège-aiguille (131) soit repoussé en arrière par le ressort (132) de telle manière que la première aiguille (121) soit toujours recouverte par le protège-aiguille (131) .

2. Un auto-injecteur (101) selon la revendication 1, comprenant en outre un mécanisme de verrouillage (135) qui empêche que le protège-aiguille (131) ne soit repoussé vers l'arrière une seconde fois.

3. Un auto-injecteur (101) selon la revendication 1 ou la revendication 2, comprenant en outre une tige indicatrice (111) qui est fixée au piston mobile (104).

4. Un auto-injecteur (101) selon la revendication 3 dans lequel la tige indicatrice (111) possède une extrémité fermée contenant à l'intérieur le moyen sollicité (112) sous forme d'un ressort, l'enveloppe externe (110) comprenant une partie transparente qui permet de suivre la progression et le statut de la progression de l'injection.

5. Un auto-injecteur (101) selon l'une des revendications précédentes dans lequel l'auto-injecteur est un auto-injecteur modulaire qui comprend trois sous-ensembles (180, 182, 183), à savoir un sous-ensemble de cartouche (181), un sous-ensemble de source d'alimentation et d'indicateur (183), et un ensemble d'aiguille et de protège-aiguille (182).

6. Un auto-injecteur (101) selon la revendication 5 dans lequel le sous-ensemble de cartouche (181) comprend un cylindre (103) et un volume de médicament (107) qui est limité par un piston (104) et un septum serti (105).

7. Un auto-injecteur (101) selon l'une des revendications 5 à 6 dans lequel le sous-ensemble de source d'alimentation et d'indicateur (183) comprend un moyen sollicité (112) et une tige indicatrice (111) qui est maintenue à l'intérieur d'une enveloppe arrière.

8. Un auto-injecteur (101) selon la revendication 7, dans lequel la tige indicatrice (111) peut être reliée au piston mobile (104) de manière à se déplacer avec lui.

9. Un auto-injecteur (101) selon l'une des revendications 6 à 8, dans lequel le moyen sollicité (112) du sous-ensemble de source d'alimentation et d'indicateur (183) est un ressort (112) qui comprend en outre un organe de verrouillage de ressort (251).

10. Un auto-injecteur (101) selon la revendication 9, dans lequel le sous-ensemble de source d'alimentation et d'indicateur (183) comprend en outre un ressort additionnel (112b) qui est moins fort que le ressort (112), pour mettre sous pression le contenu médicamenteux (107) de la cartouche avant l'injection lorsque le ressort (112) est verrouillé par l'organe de verrouillage de ressort (251).

11. Un auto-injecteur (101) selon l'une des revendications 5 à 10, dans lequel l'ensemble d'aiguille et de protège-aiguille (182) comprend une enveloppe (110b) logeant une navette à aiguilles (120) avec une aiguille d'injection (121) et une aiguille de septum (122), un protège-aiguille (131) et un ressort (132), un mécanisme de verrouillage (135) qui empêche que le protège-aiguille (131) ne soit repoussé vers l'arrière une seconde fois, et un épaulement (127) pour maintenir la cartouche (102) dans sa position correcte au moment de l'insertion.

12. Un auto-injecteur (101) selon la revendication 10 ou la revendication 11 pour ré-assemblage, dans lequel le sous-ensemble d'aiguille et de protège-aiguille (182) comprend en outre un joint (128) et un capuchon d'aiguille (126), le moyen sollicité du sous-ensemble de source d'alimentation et d'indicateur est un moteur électrique ou un réservoir de gaz liquéfié ou un ressort avec un moyen de recomprimer le ressort, et le sous-ensemble d'aiguille et de protège-aiguille (182) et le sous-ensemble de source d'alimentation et d'indicateur (183) sont reliés de manière réversible par un montage à baïonnette ou une vis, ou sont articulés l'un à l'autre.

13. Procédé pour l'assemblage d'un auto-injecteur modulaire selon la revendication 5 à 12, incluant les étapes suivantes :
insertion d'une cartouche (102) dans un sous-ensemble d'aiguille et de protège-aiguille (182), et
connexion d'un sous-ensemble de source d'alimentation et d'indicateur (183) via un moyen de connexion au sous-ensemble d'aiguille et de protège-aiguille (182) avec la cartouche (102) insérée de manière que le contenu médicamenteux de la cartouche soit mis sous pression.

14. Le processus selon la revendication 13, comprenant en outre les étapes d'insertion dans un blister ou un sachet (188) du dispositif pré-assemblé, et le passage du blister ou du sachet (188) à une stérilisation finale.

15. Le processus d'assemblage selon la revendication 14, dans lequel la stérilisation finale est effectuée par de l'oxyde d'éthylène ou du dioxyde d'azote.
